(19) Europäisches Patentamt

European Patent Office

Office européen des brevets

(11) **EP 1 294 186 A2**

(12) **EUROPEAN PATENT APPLICATION**

(43) Date of publication:
**19.03.2003 Bulletin 2003/12**

(51) Int Cl.$^7$: **H04N 5/335**, A61B 1/04

(21) Application number: **02019858.6**

(22) Date of filing: **09.09.2002**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **13.09.2001 JP 2001278645**

(71) Applicant: **Olympus Optical Co., Ltd.**
**Tokyo (JP)**

(72) Inventor: **Doguchi, Nobuyuki**
**Hino-shi, Tokyo (JP)**

(74) Representative: **von Hellfeld, Axel, Dr. Dipl.-Phys.**
**Wuesthoff & Wuesthoff**
**Patent- und Rechtsanwälte**
**Schweigerstrasse 2**
**81541 München (DE)**

(54) **Endoscope device**

(57) This invention is an endoscope device (1) which irradiates light of a plurality of specific wavelength regions, successively, onto a subject, by means of a light source device (5), and a solid-state image pickup element (14) capable of changing the charge multiplication rate receives and multiplies an optical signal on the basis of the light irradiated onto the subject, signal processing means (19) processes the output signal from the solid-state image pickup element (14), and charge multiplication rate setting means (16, 17, 18) sets the ratio of charge multiplication rates between said light of a plurality of specific wavelength regions received by the solid-state image pickup element (14).

**FIG.1**

**Description**

BACKGROUND OF THE INVENTION

1. Field of the Invention

The present invention relates to an endoscope device for obtaining images on the basis of light received from a subject by irradiating light of a plurality of specific wavelength regions onto the subject in successive fashion.

2. Description of the Related Art

Conventionally, an endoscope device for performing endoscopic examination using an endoscope having a solid-state image pickup element comprises: an endoscope, such as an electronic endoscope, a processor, light source device, and monitor. With this endoscope device, an insertable part of the endoscope is inserted into a body cavity, illumination light from the light source device is irradiated onto the subject by means of a light guide provided in the endoscope, and an output signal is obtained by photoelectric conversion of the light reflected from the subject illuminated by the illumination light, by means of a solid-state image pickup element disposed on the front end of the endoscope, this output signal being processed by the processor and then displayed on a monitor.

Moreover, in recent years, a technology has been used whereby excitation light is irradiated onto an observation region of human body tissue, the auto-fluorescence generated by the body tissue as a result of this excitation light, and the fluorescence of chemicals injected into the body, is captured as a two-dimensional image, and the state of an affection, such as cancer, or the like, in the human body (for example, the type of affection, or the degree of spreading thereof), can be diagnosed from the fluorescence image, and furthermore, fluorescence observation devices for performing fluorescence observation of this kind have been developed.

In auto-fluorescence, when excitation light is irradiated onto body tissue, fluorescence is generated on the longer wavelength side from the excitation light. Fluorescent substances in the body include: collagen, NADH (nicotine adenine dinucleotide), FMN (flavine mononucleotide), and the like. Very recently, a mutual relationship between affections and intrinsic substances in the body which create fluorescence has been revealed, and diagnosis of cancer, and the like, by means of this fluorescence, has become possible.

Moreover, in chemical fluorescence, substances such as HpD (haemato-porphyrin), Photofrin, ALA ($\delta$ - amino levulinic acid), and the like, are used as fluorescent materials which are injected into the body. These chemicals tend to accumulate in cancer regions, or the like, and therefore, by injecting them into a body and observing the fluorescence, it is possible to diagnose the cancer regions. Moreover, it is also possible to add a fluorescence substance to monoclonal antibodies, and to cause the fluorescence substance to accumulate in the affected region by means of an antigen/antibody reaction.

In view of these circumstances, Japanese Patent Application Laid-Open No. 2001-29313 presented by the present applicants discloses, as one embodiment thereof, a fluorescence observation device having variable sensitivity in the CCD disposed at the front end of an endoscope device, in such a manner that the average screen value of the fluorescence image is uniform, in other words, the monitor brightness is uniform, in order to obtain a monochromatic fluorescence image.

Moreover, Japanese Patent Laid-open No. (Hei) 10-309282 discloses a fluorescence observation device which enables the structure of organs to be observed clearly, and allows accurate diagnosis to be made, by capturing images of the fluorescent light and reflected light by means of an image intensifier attached to the outside of the scope and a variable-sensitivity CCD, and displaying these as a synthesized image.

However, supposing a case where the fluorescent light and reflected light are captured by means of the fluorescence observation device disclosed in Patent Application Laid-Open No. 2001-29313, if the CCD sensitivity is set so as to achieve a uniform average screen value in the fluorescence image, then since the fluorescent light intensity is weak compared to the reflected light intensity, the reflected light will saturate, and it will not be possible to obtain a suitable synthesized image of the fluorescent light and reflected light. Moreover, if the CCD sensitivity is set in such a manner that the average screen value of the reflected light is uniform, then since the reflected light intensity is much greater than the fluorescent light intensity, the fluorescent light will be too dark and a suitable synthesized image will not be obtained. In order to process and display the fluorescent light image and reflected light image appropriately as a synthesized image, it is necessary to obtain an image of suitable brightness, without either the fluorescent light or reflected light being saturated, or being too dark, but if there is a large disparity between the intensity of the fluorescent light and reflected light, then it is difficult to handle the images satisfactorily with the fluorescence observation device disclosed in Patent Application Laid-Open No. 2001-29313.

Moreover, in the fluorescence observation device disclosed in Japanese Patent Laid-open No. (Hei) 10-309282, it is necessary to provide a plurality of bulky and expensive image intensifiers and CCDs to correspond with the fluorescent light and reflected light created by light of a plurality of specific wavelength regions, and therefore, it is difficult to reduce the size or manufacturing costs of the device.

## SUMMARY OF THE INVENTION

It is an object of the present invention to provide an endoscope device capable of capturing images of light of a plurality of specific wavelength regions, by means of a single solid-state image pickup element, as well as capturing light of a plurality of specific wavelength regions having different intensities, respectively, as images of suitable brightness, and creating a suitable synthesized image of these images.

Briefly, the endoscope device of the present invention comprises:

> a light source device capable of irradiating light of a plurality of specific wavelength regions onto a subject, in successive fashion;
> a solid-state image pickup element for receiving an optical signal based on the light irradiated onto the subject, which is capable of changing the charge multiplication rate for multiplying this optical signal;
> signal processing means for processing the output signal from the solid-state image pickup element; and
> charge multiplication rate setting means for setting the ratio of charge multiplication rates between light of the plurality of specific wavelength regions received by the solid-state image pickup element.

The above and other objects, features and advantages of the invention will become more clearly understood from the following description referring to the accompanying drawings.

## BRIEF DESCRIPTION OF THE DRAWINGS

> Fig. 1 is a block diagram showing the general composition of an endoscope device, which is a first embodiment of the present invention;
> Fig. 2A illustrates the operation of an RGB rotating filter in special light mode in an endoscope device according to the first embodiment of the invention;
> Fig. 2B illustrates a first vertical transfer pulse supplied to the CCD in the endoscope device according to the first embodiment;
> Fig. 2C illustrates a second vertical transfer pulse supplied to the CCD in the endoscope device according to the first embodiment;
> Fig. 2D illustrates a sensitivity control pulse supplied to the CCD in the endoscope device according to the first embodiment;
> Fig. 2E illustrates a first horizontal transfer pulse supplied to the CCD in the endoscope device according to the first embodiment;
> Fig. 2F illustrates a second horizontal transfer pulse supplied to the CCD in the endoscope device according to the first embodiment;

Fig. 2G illustrates the output signal of the CCD in the endoscope device according to the first embodiment;
Fig. 3 shows the relationship between the number of sensitivity control pulses supplied to the charge multiplying mechanism CMD of the CCD and the CMD multiplication rate, in the endoscope device according to the first embodiment;
Fig. 4 shows the relationship between the intensity of the subject and the output level of the output signal in the CCD sensitivity characteristics of the endoscope device according to the first embodiment;
Fig. 5 shows the relationship between the intensity of the subject and the S/N ratio of the output signal in the CCD sensitivity characteristics of the endoscope device according to the first embodiment;
Fig. 6 is a front view showing the composition of two filter sets provided in the RGB rotating filter of the endoscope device according to the first embodiment illustrated in Fig. 1;
Fig. 7 shows the spectral characteristics of the light source device during fluorescence observation in the endoscope device according to the first embodiment illustrated in Fig. 1;
Fig. 8 illustrates the spectral characteristics of the fluorescent light and reflected light during fluorescence observation in the endoscope device according to the first embodiment illustrated in Fig. 1;
Fig. 9 shows the relationship between the number of pulses supplied to the charge multiplying mechanism CMD of the CCD, and the CMD multiplication rate, for fluorescent light, green reflected light and red reflected light, in the endoscope device according to the first embodiment illustrated in Fig. 1;
Fig. 10 is a block diagram showing the approximate composition of an endoscope device relating to the second embodiment of the present invention;
Fig. 11 is a block diagram of a CCD in the endoscope device according to the second embodiment illustrated in Fig. 10;
Fig. 12A shows the operation of an RGB rotating filter in special light mode in the endoscope device of the second embodiment illustrated in Fig. 10;
Fig. 12B illustrates vertical transfer pulses supplied to the CCD by the CCD drive means in the endoscope device according to the second embodiment illustrated in Fig. 10;
Fig. 12C illustrates a sensitivity control pulse supplied to the CCD in the endoscope device according to the second embodiment illustrated in Fig. 10;
Fig. 12D illustrates horizontal transfer pulses supplied to the CCD from the CCD drive means in the endoscope device according to the second embodiment illustrated in Fig. 10;
Fig. 12E illustrates the output signal of the CCD in the endoscope device according to the second embodiment illustrated in Fig. 10;
Fig. 13A shows an enlarged view of the time axis of the sensitivity control pulse φCMD shown in Fig. 12C, in the endoscope device according to the second embodiment illustrated in Fig. 10;

Fig. 13B shows an enlarged view of the time axis of the first vertical transfer pulse shown in Fig. 12B, in the endoscope device according to the second embodiment illustrated in Fig. 10;

Fig. 13C shows an enlarged view of the time axis of the second vertical transfer pulse shown in Fig. 12B, in the endoscope device according to the second embodiment illustrated in Fig. 10; and

Fig. 14 shows the relationship between the CMD voltage supplied to the charge multiplying mechanism CMD of the CDD, and the CMD multiplication rate, in the endoscope device according to the second embodiment illustrated in Fig. 10.

## DESCRIPTION OF THE PREFERRED EMBODIMENTS

Below, embodiments of the present invention are described with reference to the drawings.

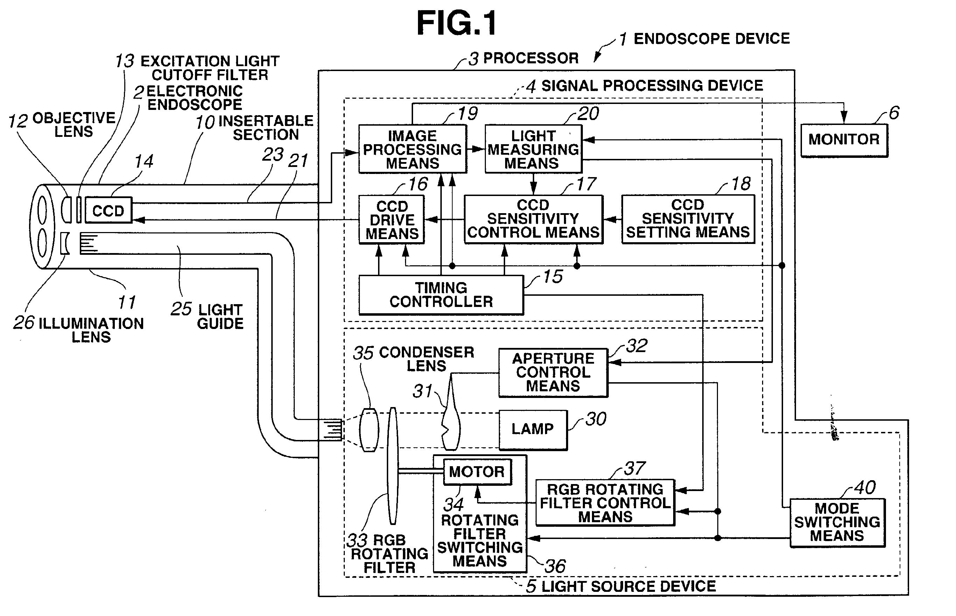

Fig. 1 is a block diagram showing the general composition of an endoscope device which is a first embodiment of the present invention.

As shown in Fig. 1, the endoscope device 1 comprises an electronic endoscope (abbreviated to "endoscope") 2 fitted with an internal solid-state image pickup element, processor 3, and monitor 6.

The processor 3 is connected freely detachably to the endoscope 2, and contains an internal signal processing device 4 and light source device 5. The monitor 6 is connected to the processor 3, and displays a video signal of which signal processing is performed by the processor 3.

The endoscope 2 comprises a long, thin insertable part 10 which is inserted inside a patient's body, an objective lens 12, an excitation light shielding filter 13, a charge coupled device (abbreviated to CCD) 14 forming a solid-state image pickup element, a light guide 25, and illumination lens 26.

The signal processor device 4 comprises a timing controller 15, CCD driving means 16, CCD sensitivity control means 17, CCD sensitivity setting means 18, image processing means 19, and light measuring means 20.

The light source device 5 comprises a lamp 30, aperture 31, aperture control means 32, RGB rotating filter 33, motor 34, condensing lens 35, rotating filter switching means 36, RGB rotating filter control means 37, and mode switching means 40. The light source device 5 is capable of irradiating light of a plurality of specific wavelength regions onto the subject, in successive fashion.

The CCD 14 forms a solid-state image pickup element capable of receiving an optical signal based on the light irradiated onto the subject, and changing the charge amplification rate by which this optical signal is amplified.

The image processing means 19 is signal processing means for processing the output signal from the CCD 14.

The CCD driving means 16, CCD sensitivity control means 17 and CCD sensitivity setting means 18 is charge multiplication rate setting means for setting the ratio between the charge multiplication rates for the light of a plurality of specific wavelength regions received by the CCD 14.

The CCD 14 has a charge multiplying mechanism CMD (charge multiplying detector), and by applying a pulsed high-field drive signal to the charge multiplying mechanism CMD (charge multiplying detector), the aforementioned charge multiplication rate can be changed. The charge multiplication rate setting means sets the ratio of the aforementioned charge multiplication rates of the CCD 14, by changing the number of pulses of the aforementioned pulsed high-field drive signal.

Next, the endoscope 2 will be described in detail.

The objective lens 12, excitation light shielding filter 13, CCD 14, front end of the light guide 25, and irradiation lens 26 are contained in the front end section 11 of the insertable part 10 of the endoscope 2.

The light guide 25 comprises a bundle of optical fibres which propagate illumination light. The illumination lens 26 is provided on the front end side of the light guide 25.

Illumination light from the light source device 5 having respective spectral characteristics is transmitted inside the endoscope 2 by the light guide 25, and is successively irradiated onto the subject via the illumination lens 26.

The objective lens 12 forms an image of the subject, such as body tissue, or the like, onto which the illumination light is irradiated by the illumination lens 26. The light receiving surface of the CCD 14 is disposed at the focal point of the objective lens 12. An excitation light shielding filter 13 which only transmits certain specific wavelength regions is provided on the front side of the CCD 14. This excitation light shielding filter 13 has light splitting characteristics whereby, for example, it transmits the waveband of the auto-fluorescence generated by the body tissue, but does not transmit the excitation light.

The CCD 14 is connected to the CCD drive means 16 provided in the signal processor device 4 in the processor 3, by means of a drive signal line 21, and it is also connected to the image processing means 19 provided in the processor 3, by means of a CCD output signal line 23.

The CCD 14 exposes the light receiving surface, controls the sensitivity thereof, and performs read out, in accordance with the drive signals generated by the CCD drive means 16, and the output signal read out thereby is supplied to the image processing means 19.

Thereby, an image of the subject formed on the light receiving surface of the CCD 14 via the objective lens 12 and excitation light shielding filter 13 is converted to an electrical signal by the CCD 14, and this output signal is supplied to the image processing means 19.

Here, the CCD 14 used is that described in "US. PAT. No. 5,337,340 "charge multiplying detector (CMD) suitable for small pixel CCD image sensors", the contents of which are hereby incorporated by reference". In other words, the CCD 14 is provided with a charge multiplying mechanism CMD (charge multiplying detector) for each pixel, or between the horizontal register and the detector amp.

In this CCD 14, when a pulsed voltage of high-field (energy of approximately 1.5 times the energy gap) is applied to the charge multiplying mechanism CMD, then the signal charge receives energy from the strong electric field, due to the charge multiplying mechanism CMD, and collides with the electrons in the valence band, and a new charge is generated due to the ionization created by this collision. The charge multiplying mechanism CMD is able to multiply the number of signal charges as desired, by successively applying a plurality of pulses having a certain strength (amplitude) and thereby generating successive ionizing collisions to create successive signal charges.

The CCD 14 of the present embodiment uses a monochromatic CCD fitted with a charge multiplying mechanism CMD for each pixel.

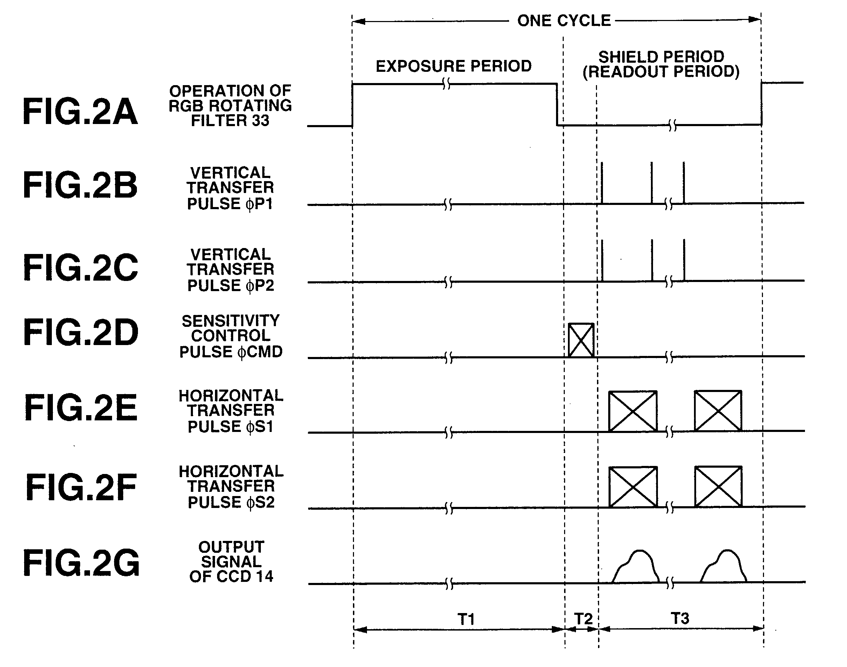

Next, the drive signals supplied to the CCD 14 by the CCD drive means 16 are described with reference to Fig. 2A to Fig. 2G. These diagrams are timing charts showing the relationship between the drive signal and the CCD output signal in special light mode.

Fig. 2A shows the operation of the RGB rotating filter 33 in special light mode in the endoscope device according to the first embodiment of the invention; Fig. 2B illustrates a first vertical transfer pulse φP1 supplied to the CCD 14 in the endoscope device according to the first embodiment; Fig. 2C illustrates a second vertical transfer pulse φP2 supplied to the CCD 14 in the endoscope device according to the first embodiment; Fig. 2D illustrates a sensitivity control pulse φCMD supplied to the CCD 14 in the endoscope device according to the first embodiment; Fig. 2E illustrates a first horizontal transfer pulse φS1 supplied to the CCD 14 in the endoscope device according to the first embodiment; Fig. 2F illustrates a first horizontal transfer pulse φS2 supplied to the CCD 14 in the endoscope device according to the first embodiment; and Fig. 2G illustrates the output signal of the CCD 14 in the endoscope device according to the first embodiment.

As shown in Fig. 2A, in the special light mode, the RGB rotating filter 33 performs one cycle with the exposure time (T1) and shield time (T2 + T3).

The time period T1 is the exposure time during which light from the lamp 30 is transmitted into the endoscope 2. During this period T1, the CCD 14 accumulates the light incident on the light receiving surface thereof from the subject, in the form of signal charge by photoelectric conversion.

The shield period (T2 + T3) is the time period during which the light from the lamp 30 is shut out and read out from the CCD 14 is performed.

During T2, the CCD drive means 16 supplies a plurality of sensitivity control pulses φCMD, as illustrated in Fig. 2D, to the charge multiplying mechanism CMD of the CCD 14. Thereby, during period T2, the signal charge accumulated during period T1 is multiplied by the charge multiplying mechanism CMD.

During period T3, the CCD 14 transfers the signal charge for one horizontal line to a horizontal register, by means of the first and second vertical transfer pulses φP1, φP2 shown in Fig. 2B and Fig. 2C, and the signal charge transferred to the horizontal register is then transferred successively to the output amp section, by means of the first and second horizontal transfer pulses φS1, φS2, shown in Fig. 2E and Fig. 2F. The output amp section of the CCD 14 converts the transferred charge to a voltage and outputs this voltage as the output signal of the CCD 14 as shown in Fig. 2G.

Thereby, the CCD 14 is able to achieve a prescribed sensitivity (CMD multiplication rate) by changing the number of sensitivity control pulses φCMD sent to the charge multiplying mechanism CMD by the CCD drive means 16.

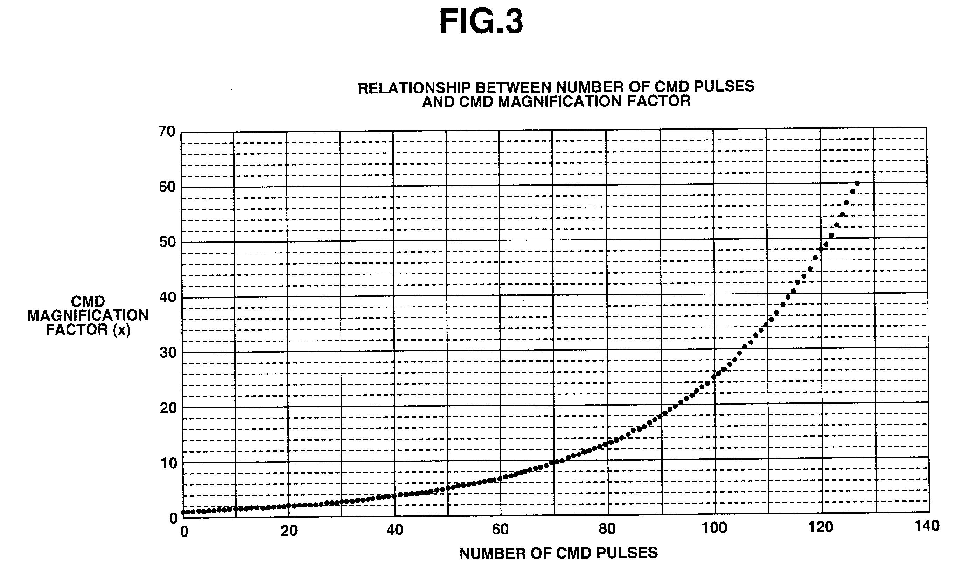

Next, the relationship between the number of pulses supplied to the charge multiplying mechanism CMD of the CCD 14 and the CMD multiplication rate (signal charge multiplication rate in the CMD) will be described with reference to Fig. 3.

As shown in Fig. 3, the multiplication rate of the charge multiplying mechanism CMD increases exponentially in proportion to the number of pulses supplied to the charge multiplying mechanism CMD.

The gradient of the exponential function changes according to the magnitude of the pulse voltage supplied to the charge multiplying mechanism CMD, and if the voltage is increased, then a smaller number of pulses is required to obtain the same CMD multiplication rate, whereas if the voltage is reduced, then a larger number of pulses is required to obtain the same CMD multiplication rate.

In normal light mode, no sensitivity control pulses φCMD are output to the CCD 14 during period T2.

When the number of sensitivity control pulses φCMD is zero, the CMD multiplication rate is one (no multiplication), and the characteristics are the same as standard CCD sensitivity. In normal light mode, it is also possible to output a predetermined number of sensitivity control pulses φCMD to the CCD 14.

The timing controller 15 is connected to the CCD drive means 16, the CCD sensitivity control means 17, the image processing means 19, and the RGB rotating filter control means 37, and synchronizes these elements.

The image processing means 19 is devised in such a manner that it operates differently in normal light mode and special light mode, in accordance with a mode switching signal from the mode switching means 40.

In normal light mode, the image processing means 19 converts output signals from the CCD 14 into television signals and outputs the same to recording means and display means such as monitor 6 and also outputs luminance signals to the light measuring means 20.

In normal light mode, three wavelengths (spectral characteristics of red R1, green G1, and blue B1) from the CCD 14 have different white balance coefficients, because the image processing means 19 performs white balance correction on these three wavelengths.

In special light mode (fluorescence observation), the image processing means 19 performs image calculation and processing of the output signal corresponding to the three wavelengths from the CCD 14, for the purpose of fluorescence observation, and outputs the resulting signal to display means, such as the monitor 6, and recording means. The image processing means 19 outputs the average screen value of a monochromatic image corresponding to the respective wavelengths, to the light measuring means 20.

In special light mode, the image processing means 19 switches the white balance coefficient for the 3 wavelengths (spectral characteristics of Ex1, Ex2, Ex3) to a white balance coefficient for fluorescence observation. This white balance coefficient for fluorescence observation can be set to any coefficient, but in the example of the present embodiment, the white balance coefficient $kEx1 : kEx2 : kEx3$ corresponding to Ex1, Ex2, Ex3 is set to 1:1:1.

The light measuring means 20 is connected to the post-step of the image processing means 19 and performs different operation in the normal light mode and special light mode, in accordance with a mode switching signal from the mode switching means 40.

In normal light mode, a luminance signal processed by the image processing means 19 is input from the image processing means 19 to the light measuring means 20.

The light measuring means 20 compares this luminance signal with a monitor brightness value set as desired by an operator, and it outputs the comparison result to aperture control means 32. The aperture control means 32 controls the opening and closing of the aperture 31 on the basis of the comparison information (information indicating which is larger).

In special light mode, the monochromatic light of three wavelengths (fluorescent light, reflected light in the green narrow band, and reflected light in the red narrow band), passed respectively by the Ex1, Ex2 and Ex3 filters of the RGB rotating filter 33, is captured at different timings by the CCD 14, and the output signal image processing means 19 inputs the output signals corresponding respectively to the three wavelengths of monochromatic light from the CCD 14, to the light measuring means 20. The light measuring means 20 respectively calculates the average screen value for the luminosity of the monochromatic images corresponding to each of the three wavelengths of monochromatic light, and output the average screen value for a predetermined monochromatic image of the aforementioned monochromatic images of three wavelengths, to the CCD sensitivity control means 17.

In this embodiment, as an example, it is supposed that the average screen value of the fluorescent image, is set. However, the predetermined monochromatic image may be the reflected light or a synthesized image of the fluorescent light and the reflected light.

The CCD sensitivity control means 17 operates in accordance with the mode switching signal from the mode switching means 40 and only functions in special light mode (fluorescence observation).

The main functions of the CCD sensitivity control means 17 are two: a CMD-AGC function and a CMD multiplication rate uniform ratio control function.

The CMD-AGC (auto gain control) function is a function for controlling the multiplication rate of the charge multiplying mechanism CMD, in such a manner that the output signal level from the CCD 14 is uniform with respect to change in the light intensity incident on the light receiving surface of the CCD 14.

The CMD multiplication rate uniform ratio control function is a function for controlling the ratio between the CMD multiplication rates for the three wavelengths to a uniform ratio, when capturing images corresponding to the Ex1, Ex2 and Ex3 filters of the RGB rotating filter 33.

Below, the CMD-AGC function of the CCD sensitivity control means 17 is described in detail.

When exercising the CMD-AGC function, the CCD sensitivity control means 17 inputs the average screen value of the monochromatic image of the predetermined wavelength (in the present embodiment, the fluorescent light image), from the light measuring means 20, and compares this average screen value with the monitor brightness value set as desired by the operator. The CCD sensitivity control means 17 then calculates the number of sensitivity control pulses $\phi CMD$ to output to the charge multiplying mechanism CMD of the CCD 14, from the comparison result (magnitude relationship), and it outputs this number of pulses to the CCD drive means 16.

The calculation formula for the number of pulses used by the CCD sensitivity control means 17 in this case is described below.

In the charge multiplying mechanism CMD of the CCD 14, the relationship between the number of sensitivity control pulses $\phi CMD$ (CMD pulses) and the CMD multiplication rate illustrated in Fig. 3 is expressed by Equation 1 below.

$$A(p) = C \cdot \mathrm{Exp}(\alpha p) \qquad (1)$$

where A(p) is the CMD multiplication rate when the number of sensitivity control pulses φCMD is p, and C and α are constants (intrinsic device constants).

According to these characteristics, when imaging a subject of a certain intensity, the average screen value of the output image from the CCD 14 obtained by increasing or reducing the number of sensitivity control pulses φCMD changes in an exponential fashion.

An up/down counter (not illustrated) is provided in the CCD sensitivity control means 17. This up/down counter comprises a memory, which records the number of sensitivity control pulses φCMD output to the charge multiplying mechanism CMD with respect to a reference wavelength (in the present embodiment, the fluorescent light image).

The CCD sensitivity control means 17 compares the average screen value of the fluorescent light image with the monitor brightness set by the operator, and if the fluorescent light image is dimmer than the monitor brightness set by the operator, then the number of sensitivity control pulses φCMD, p, is incremented by 1 pulse, by the up/down counter, in order to increase the multiplication rate of the charge multiplying mechanism CMD, and at the next fluorescence image capturing timing, (p+1) pulses are output to the CCD drive means 16. Conversely, if the fluorescent light image is brighter than the monitor brightness set by the operator, then the CCD sensitivity control means 17 decrements the number of sensitivity control pulses φCMD, p, by one pulse, and at the next fluorescence image capturing timing, (p-1) pulses are output to the CCD drive means 16. The newly output number of sensitivity control pulses φCMD is written over the memory of the up/down counter, and stored therein.

In this way, the CCD sensitivity control means 17 increases and decreases the number of sensitivity control pulses φCMD, on the basis of a comparison between the average screen value of the fluorescent light image and the monitor brightness set by the operator, in such a manner that the average screen value of the fluorescent light image matches the monitor brightness set by the operator, in response to increase or decrease in the intensity of the fluorescent light from the subject.

Moreover, the CCD sensitivity control means 17 can be set to have a maximum limit and minimum limit for the number of sensitivity control pulses φCMD, in such a manner that it outputs a number of sensitivity control pulses φCMD within this range, to the CCD drive means 16.

Below, the uniform ratio control function for the CMD multiplication rate of the CCD sensitivity control means 17 is described in detail.

When exercising the CMD multiplication rate uniform ratio control function, the CCD sensitivity control means 17 respectively inputs the numbers of pulses (differentials) corresponding to the ratio of the CMD multiplication rates required for the two remaining wavelengths, on the basis of the CMD multiplication rate set for the reference wavelength (in the present embodiment, the fluorescent light image) from the CCD sensitivity setting means 18.

The memory in the up/down counter of the CCD sensitivity control means 17 stores the number of sensitivity control pulses φCMD output to the charge multiplying mechanism CMD for the reference wavelength.

The CCD sensitivity control means 17 is provided with pulse number calculating means (not illustrated), which adds or subtracts the number of pulses input from the CCD sensitivity setting means 18, to or from the number of sensitivity control pulses φCMD for the reference wavelength stored in the memory of the up/down counter.

In synchronization with the timings for capturing the reflected light images (two images : one in the green narrow band and one in the red narrow band), the CCD sensitivity control means 17 respectively adds or subtracts the number of pulses input from the CCD sensitivity setting means 18, to or from the number of sensitivity control pulses φCMD output to the CCD drive means 16 during capturing the image of fluorescent light, by means of the pulse number calculating means, and outputs the result to the CCD drive means 16.

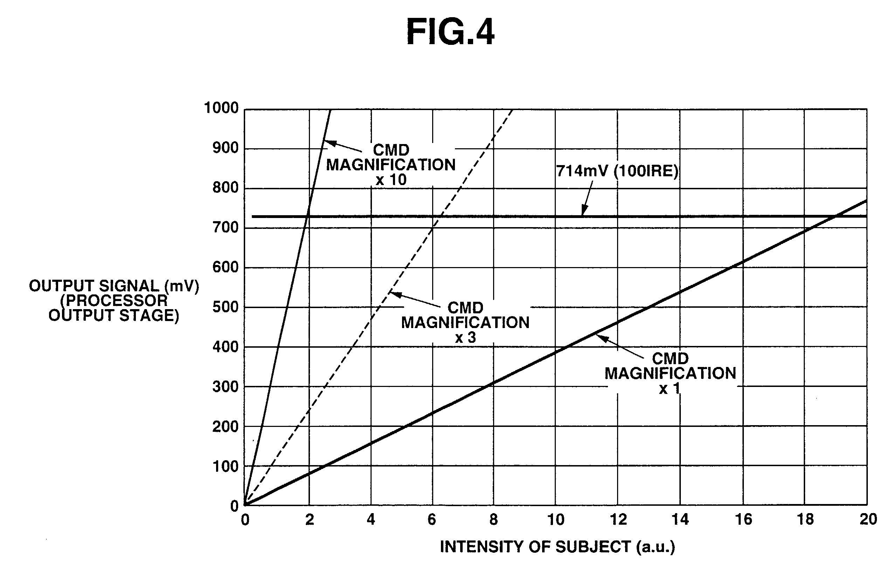

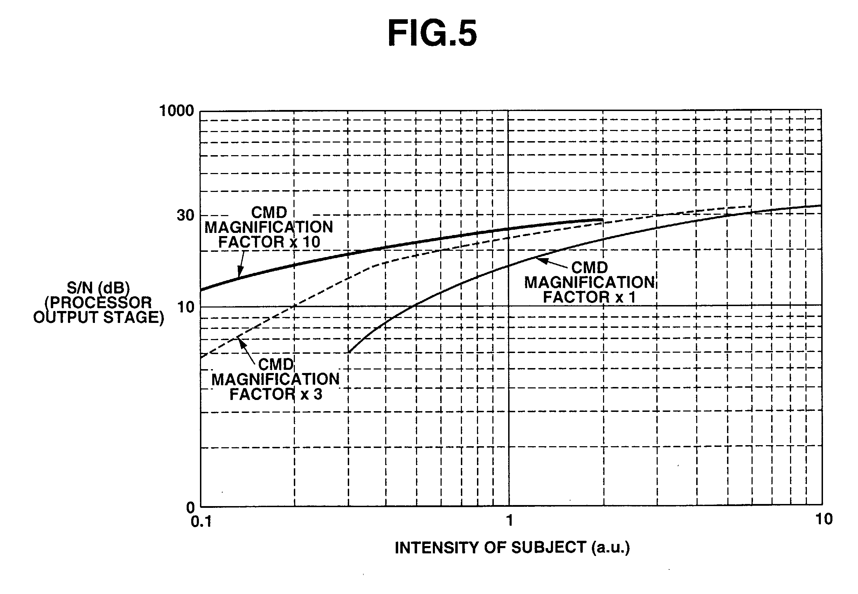

Next, with reference to Fig. 4 and Fig. 5, a description is given of the signal output level and signal-to-noise ratio S/N corresponding to the subject intensity at the output stage of the processor 3, when the number of pulses input to the charge multiplying mechanism CMD is changed, thereby changing the CMD multiplication rate.

Fig. 4 shows the relationship between the subject intensity and the output level of the output signal, in relation to the CCD sensitivity characteristics of the endoscope device according to the first embodiment illustrated in Fig. 1. Fig. 5 shows the relationship between the subject intensity and the output signal S/N ratio in relation to the CCD sensitivity characteristics of the endoscope device according to the first embodiment illustrated in Fig. 1.

As shown in Fig. 4, the level of the output signal at the output stage of the processor 3 becomes larger with respect to the subject intensity, the greater the CMD multiplication rate.

Furthermore, as shown in Fig. 5, the signal-to-noise ratio S/N of the output signal at the output stage of the processor 3 becomes larger with respect to the subject intensity, the greater the CMD multiplication rate. The differential with respect to a CMD multiplication rate of 1 (no multiplication) increases as the subject intensity decreases.

Therefore, in very weak regions (where the subject intensity is low), at a CMD multiplication rate of 1 (no multiplication), a low quality image, which is dark and contains a large amount of noise, will be displayed on the monitor, but as the CMD multiplication rate increases, an image of higher quality, which is brighter and contains less noise, is obtained on the monitor 6.

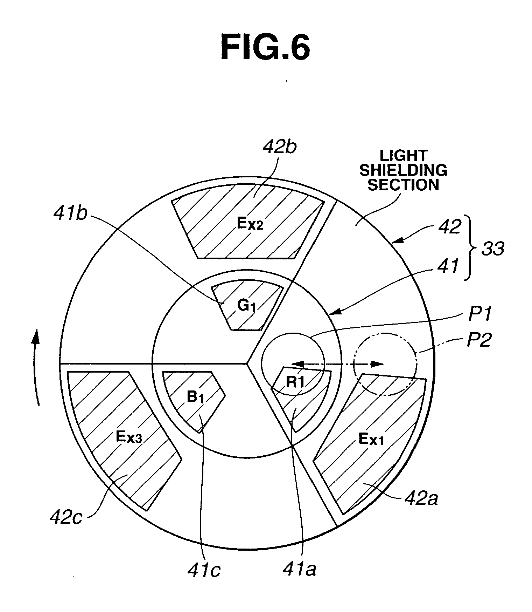

Next, the CCD sensitivity setting means 18 is described in detail with reference to Fig. 6.

Fig. 6 is a front view showing the composition of two filter sets provided in the RGB rotating filter 33 in Fig. 1.

The CCD sensitivity setting means 18 in Fig. 1 forms means for controlling the ratio of the CMD multiplication rates used when obtaining respective images corresponding to the three wavelengths (Ex1, Ex2, Ex3) of the RGB rotating filter 33 in Fig. 6, during special light mode (fluorescence observation).

The CCD sensitivity setting means 18 comprises two sets of setting means, consisting of DIP switches or rotary DIP switches, or the like, which respectively set arbitrarily the number of pulses (differentials) corresponding to the ratio of the CMD multiplication rates desired for the two remaining wavelengths, on the basis of the CMD multiplication rate for the reference wavelength (in the present embodiment, the fluorescent light image), and output the result to the CCD sensitivity control means 17.

The method used to set the number of pulses (differentials) in this case is described below.

Here, the relationship between the number of sensitivity control pulses φCMD and the CMD multiplication rate in the charge multiplying mechanism CMD of the CCD 14 is expressed by Equation (1) above, when the number of sensitivity control pulses φCMD is taken as p.

If the number of sensitivity control pulses φCMD is (p - k), then Equation (2) is obtained.

$$A(p-k) = C \cdot Exp\{\alpha(p-k)\} \qquad (2)$$

From Equations (1) and (2), the ratio of the CMD multiplication rates in a case where the numbers of pulses are p pulses and (p-k) pulses will be expressed by Equation (3).

$$A(p-k) / A(p) = C \cdot Exp\{\alpha(p-k)\} / C \cdot Exp(\alpha p)$$

$$= Exp(-\alpha k) \qquad (3)$$

From Equation (3), if it is wished to maintain a uniform ratio for the CMD multiplication rate of the reflected light, with respect to the CMD multiplication rate for the reference fluorescent light image, then the CCD sensitivity setting means 18 should subtract the number of pulses k corresponding to Exp(-αk) from the number of sensitivity control pulses φCMD corresponding to the fluorescent light image. When the CCD sensitivity setting means 18 decreases the number of pulses, a CMD multiplication rate which is smaller than the reference CMD multiplication rate is obtained, and conversely, when it increases the number of pulses, a CMD multiplication rate which is larger than the reference CMD multiplication rate is obtained. The CCD sensitivity setting means 18 can be set to any initial value for the number of sensitivity control pulses φCMD.

The mode switching means 40 forms a switch whereby the operator can select, as he or she desires, between different observation modes, namely, a normal light mode (normal light observation), and a special light mode (fluorescence observation).

In the case of the present embodiment, the mode switching means 40 is provided in the processor 3, but it may be provided in the endoscope 2.

The mode switching means 40 is connected to the rotating filter switching means 36, RGB rotating filter control means 37, aperture control means 32, light measuring means 20, CCD drive means 16, CCD sensitivity control means 17 and image processing means 19, and it outputs a mode switching control signal corresponding to the observation mode to each of these means.

Each of the means connected to the mode switching means 40 performs operations corresponding to the observation mode.

Next, the constituent elements of the light source device 5, namely, the lamp 30, aperture 31, aperture control means 32, RGB rotating filter 33, motor 34, condensing lens 35, RGB rotating filter switching means 36 and RGB rotating filter control means 37, are described.

The lamp 30 generates illumination light.

The condensing lens 35 condenses the rays of illumination light from the lamp 30 onto the back end face of the light guide 25.

The RGB rotating filter 33 is inserted between the lamp 30 and the condensing lens 35. The RGB rotating filter 33 is connected rotatably to the shaft of a motor 34.

The RGB rotating filter control means 37 is capable of controlling the rotational speed of the RGB rotating filter 33 and motor 34, as desired, according to the observation mode. For example, the RGB rotating filter control means 37 is able to slow down the rotational speed of the motor 34 in the special light mode, thereby increasing the CCD 14 exposure time to be larger time compared to normal light mode.

The aperture 31 is inserted between the lamp 30 and RGB rotating filter 33.

Opening and closing of the aperture 31 is controlled by the aperture control means 32, thereby controlling the amount of light falling on the back end face of the light guide 25.

The opening of the aperture 31 is maintained at different positions in special light mode and normal light mode.

The RGB rotating filter 33 has a dual structure comprising two sets of filters 41, 42, on the inner circumference portion and outer circumference portion thereof, as illustrated in Fig. 6.

The first filter set 41 in the inner circumference portion is constituted by three filters 41a, 41b, 41c having red (R1), green (G1) and blue (B1) spectral characteristics for normal light mode.

The second filter set 42 in the outer circumference portion is constituted by three filters 42a, 42b, 42c having spectral characteristics Ex1, Ex2, Ex3 for special light mode (fluorescence observation).

The filter 42a of spectral characteristics Ex1 is an excitation light filter for transmitting the light in 390 - 470 nm region.

The filter 42b of spectral characteristics Ex2 is a reflected light filter having spectral characteristics of a narrow band of approximately 10 nm half-width and a central wavelength in the vicinity of 550 nm, and a transmission factor of several percent (%), approximately.

The filter 42c of spectral characteristics Ex3 is a reflected light filter having spectral characteristics of a narrow band of approximately 10 nm half-width and a central wavelength in the vicinity of 600 nm, and a transmission factor of several percent (%), approximately.

Shielding sections are formed between the filters 41a, 41b and 41c. These shielding sections corresponding to shield period (readout period) of the CCD 14. Similar shield sections are provided between the filters 42a, 42b and 42c in the second filter set 42.

The RGB rotating filter switching means 36 is installed in such a manner that it can move the motor 34, thereby causing the whole RGB rotating filter 33 to move in such a manner that the inner circumference side or outer circumference side thereof can be placed selectively in the axis of the illumination light. In this case, the RGB rotating filter switching means 36 selectively moves the first filter set 41 on the inner circumference side and the second filter set 42 on the outer circumference side, onto the axis of the illumination light linking the lamp 30 to the back end face of the light guide 25, and thereby performs switching between the first filter set 41 and second filter set 42.

In normal light mode, the light beam P1 (solid line in Fig. 6) from the lamp 30 is directed onto the inner side filter set 41.

In special light mode, the light beam P2 (broken line in Fig. 6) from the lamp 30 is directed onto the outer side filter set 42.

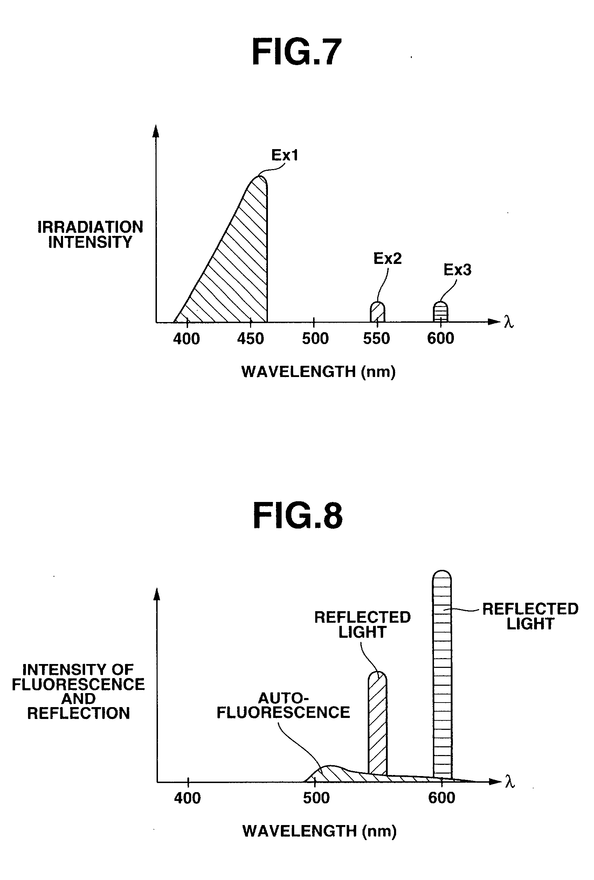

By means of this composition, the illumination light irradiated from the illumination lens 26 of the endoscope 2 in special light mode has the spectral characteristics shown in Fig. 7.

Fig. 7 shows the spectral characteristics of the light source device during fluorescence observation in the endoscope device 1 shown in Fig. 1, and Fig. 8 illustrates the spectral characteristics of the fluorescent light and reflected light during fluorescence observation in the endoscope device 1.

The action in the normal light mode and special light mode (fluorescence observation) according to the present embodiment are described below.

To perform observation, the operator inserts the insertable part 10 of the endoscope 2 shown in Fig. 1 into the body cavity of the patient (windpipe, oesophagus, stomach, colon, or the like).

In normal light mode (normal light observation, the first filter set 41 of the rotating filter 33 shown in Fig. 6 is disposed in the illumination light path, and the CMD multiplication rate of the CCD 14 is set to 1. The illumination light irradiated from the lamp 30 is passed through the first filter set 41, whereby illumination light passing the R (red), G (green), B (blue) faces is successively irradiated from the illumination lens 26 on the endoscope 2, onto the body tissue.

The reflected light from the body tissue corresponding to the successive R, G, B illumination light is incident successively on the CCD 14, and output signals corresponding to the R, G, B are input to the image processing means 19, whereby a normal light observation image is displayed on the monitor 6.

In the light measuring means 20, the luminance signal for one screen is compared with the monitor brightness reference value set by the operator, and the comparison result is output to the aperture control means 32. This aperture control means 32 controls the opening and closing of the aperture 31 in accordance with the comparison results.

In the comparison made by the light measuring means 20, if the subject is brighter than the set reference value, then the aperture control means 32 causes the aperture 31 to operate in such a manner that it closes (the irradiation intensity on the back end face of the light guide reduces).

On the other hand, if, in the comparison made by the light measuring means 20, the subject is darker than the set reference value, then the aperture control means 32 causes the aperture 31 to operate in such a manner that it opens (the irradiation intensity on the back end face of the light guide increases).

In this way, by opening and closing the aperture 31 to alter the irradiation intensity on the body tissue, automatic light adjustment is performed whereby the brightness of the monitor 6 maintains the value set by the operator.

The reference value set for brightness of the motor 6 may be set by the operator to any desired brightness by, for example, connecting a keyboard, or other input means, to the light measuring means 20.

In Fig. 4 and Fig. 5, the respective characteristics of the output signal and S/N ratio in the subject image displayed on the monitor 6 correspond to a CMD multiplication rate of 1.

In the case of special light mode (special light observation: fluorescence observation), the operator selects the special light mode (fluorescence observation) by means of a mode switch constituting mode switching means 40. Thereby, the rotating filter switching means 36 operate and the second filter set 42 of the rotating filter 33 shown in Fig. 6 is disposed in the illumination light path, whilst the aperture 31 is held at a fully open position, for example, in such a manner that strong illumination light is incident on the back end face of the light guide 25.

The illumination light irradiated from the lamp 30 passes through the respective filters 42a, 42b, 42c of the second filter set 42 of the RGB rotating filter 33, which rotates, whereby excitation light in a blue region corresponding to spectral characteristics Ex1, green narrow band light corresponding to spectral characteristics Ex2, and red narrow band light corresponding to spectral characteristics Ex3 are formed successively, and input to the back end face of the light guide 25, via the condensing lens 35. Thereby, illumination light having the spectral characteristics shown in Fig. 7 is irradiated successively (intermittently) onto the body tissue, from the illumination lens 26 disposed at the front end of the endoscope 2.

When excitation light having spectral characteristics Ex1 is irradiated onto the body tissue, the reflected light of the actual excitation light, and auto-fluorescence light generated by the body tissue as a result of the excitation light (caused, for instance, by NADH, Flavine, or the like), is input to the objective lens 12. The reflected excitation light contained in the light transmitted by the objective lens 12 is cut out by the excitation light shielding filter 13, and the auto-fluorescence light is input to the light receiving section of the CCD 14. Moreover, when the green narrow band and red narrow band light according to spectral characteristics Ex2 and Ex3 is irradiated onto the body tissue, the green narrow band light and red narrow band light that is reflected by the body tissue is input to the objective lens 12, and the light transmitted by the objective lens 12 is then passed through an excitation light shielding filter 13 and input to the light receiving surface of CCD 14.

In this way, the fluorescent light, reflected green light (green narrow band) and reflected red light (red narrow band) from the body tissue are input successively to the CCD 14.

The output signal from the CCD 14 corresponding to the respective wavelengths is input to the image processing means 19, which performs image processing for fluorescence observation and displays a fluorescence observation image on the monitor 6.

In the image processing means 19, the white balance coefficient when capturing images of the three wavelengths, namely, the fluorescent light, green reflected light and red reflected light, is set, for example, to fluorescent light : green reflected light : red reflected light = 1:1:1, for special light mode (fluorescence observation).

The intensity of the auto-fluorescence generated by the spectral characteristics Ex1 is very weak compared to the intensity of the reflected light, and therefore, when the illumination light according to the spectral characteristics Ex1, Ex2, Ex3 as shown in Fig. 7 is irradiated onto healthy body tissue, auto-fluorescence and reflected light (green narrow band, red narrow band) spectra somewhat like those illustrated in Fig. 8 will be obtained at the light receiving surface of the CCD 14. Here, the intensity ratio between the respective spectra is approximately fluorescent light : green reflected light (green narrow band) : red reflected light (red narrow band) = 1 : 5 : 10.

Below, the process of setting the ratio of the CMD multiplication rates for the auto-fluorescence and the reflected light (green narrow band, red narrow band) is described in detail.

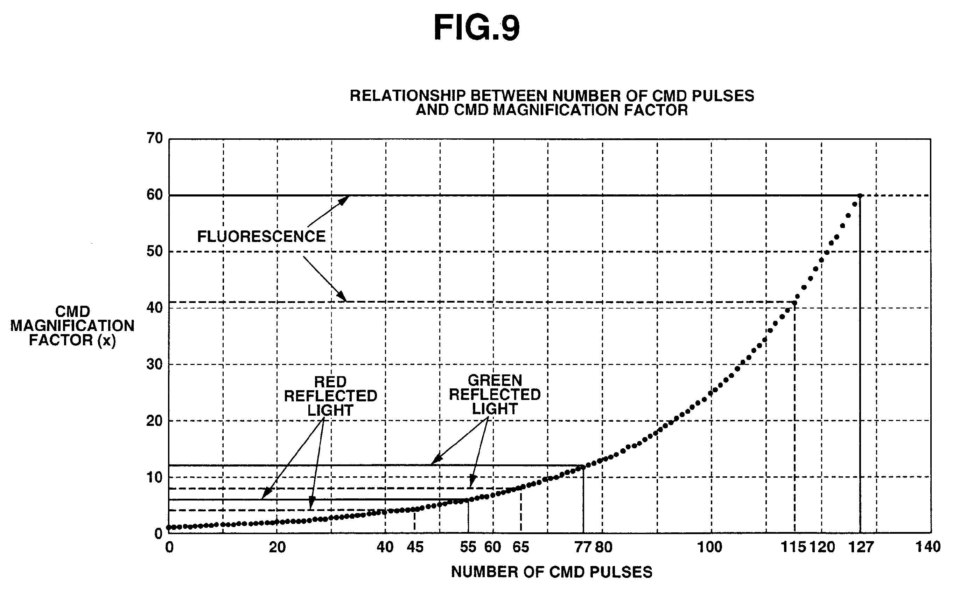

Fig. 9 shows the relationship between the number of pulses supplied to the charge multiplying mechanism CMD of the CCD 14, and the CMD multiplication rate, for fluorescent light, green reflected light and red reflected light.

Here, for example, if the CCD 14 successively receives fluorescent light, green reflected light and red reflected light obtained from healthy tissue, when the illumination light in Fig. 8 is irradiated onto healthy tissue, then the CCD sensitivity setting means 18 determines the ratio of the CMD multiplication rates for each wavelength, and calculates the number of pulses corresponding to that ratio, in such a manner that the output signal levels from the CCD 14 corresponding respectively to the fluorescent light, green reflected light and red reflected light are the same.

In Fig. 8, the intensity ratio between the spectra of different wavelengths is fluorescent light : green reflected light : red reflected light = 1 : 5 : 10. Therefore, the ratio of the CMD amplification rates for each wavelength will be fluorescent light : green reflected light : red reflected light = 10: 2 : 1.

When images of the subject (body tissue) are captured, if the ratio determined above is maintained for the CMD multiplication rates of the respective wavelengths, then the output signal of the CCD 14 will have an approximately uniform level.

For example, supposing that a reference CMD multiplication rate of 60 times is set, then in order to obtain a CMD multiplication rate of 60 times, the number of CMD input pulses will be 127 pulses. Since the ratio of the CMD multiplication rates for each wavelength is fluorescent light : green reflected light : red reflected light = 10 : 2 : 1, then if the CMD multiplication rate for fluorescent light is 60 times, the respective CMD multiplication rates for fluorescent light : green reflected light : red reflected light will be 60 : 12 : 6.

Accordingly, the number of CMD input pulses required for achieving respective CMD multiplication rates for the green reflected light and red reflected light of 12 times and 6 times, will be 77 pulses and 55 pulses.

Therefore, taking the number of pulses for obtaining the CMD multiplication rate required for capturing illumination light as a reference value, the number of CMD input pulses for capturing images of the green reflected light and red reflected light is calculated by the pulse number calculating means by subtracting 50 pulses (= 127 - 77) and 72 pulses (= 127 - 55) from the number of CMD input pulses for capturing images of the fluorescent light. In this case, the numbers of pulses (differentials) corresponding to the desired ratio of CMD multiplication rates for the green reflected light and red reflected light, with respect to the CMD multiplication rate for the reference wavelength (in this embodiment, the fluorescent light image), are set respectively to 50 pulses and 72 pulses in the CCD sensitivity setting means 18.

The CMD-AGC operation is now described.

The light measuring means 20 calculates the average value of the output signal from the CCD 14 for one screen of a reference fluorescent light image, and output this average to the CCD sensitivity control means 17.

The CCD sensitivity control means 17 compares the average screen value of the fluorescence image with the prescribed monitor brightness set by the operator. The brightness setting for the fluorescent light image on the monitor 6 can be set to any brightness by the operator, by connecting a keyboard, switch, or other input means to the CCD sensitivity control means 17, for example. In the comparison in the CCD sensitivity control means 17, if the fluorescent light image is dimmer than the value set by the operator, then the CCD sensitivity control means 17 increments the number of CMD input pulses by one pulse from the current number, in order to increase the multiplication rate of the charge multiplying mechanism CMD, and at the next fluorescence image capturing timing, this new pulse number is output to the charge multiplying mechanism CMD of the CCD 14, via the CCD drive means 16. Conversely, if, in the comparison in the CCD sensitivity control means 17, the fluorescent light image is brighter than the value set by the operator, then the CCD sensitivity control means 17 decrements the number of CMD input pulses by one pulse, and at the next fluorescence image capturing timing, this new pulse number is output to the charge multiplying mechanism CMD of the CCD 14, via the CCD drive means 16.

Therefore, the CCD sensitivity control means 17 performs auto gain control whereby, if the output signal from the processor 3 is smaller than the value set by the operator, then the CMD multiplication rate in the CCD 14 is increased, and conversely, if the output signal from the processor 3 is larger than the value set by the operator, then the CMD multiplication rate of the CCD 14 is reduced.

A concrete example of an operation for uniform control of the ratio of CMD multiplication rates is described below.

At the image capturing timing for the green reflected light and red reflected light, the CCD sensitivity control means 17 subtracts 50 pulses and 72 pulses, respectively, which were input (set) in the CCD sensitivity setting means 18, from the number of input pulses which were input to the charge multiplying mechanism CMD of the CCD 14 when capturing the fluorescent light image, and outputs these numbers of pulses to the charge multiplying mechanism CMD of the CCD 14 via the drive means 16.

As an example, a case where an operator views the interior of a patient's body cavity in special light mode (fluorescence observation) shall be considered.

Here, a case is supposed where the endoscope 2 observes the subject, or body tissue, at a distant point therefrom, and it is assumed that the CMD multiplication rate required to obtain a monochromatic image of fluorescent light by auto gain control is 60 times (number of CMD input pulses = 127 pulses).

If the CCD 14 captures the fluorescent light images at a CMD multiplication rate of 60 times, then the synthesized image processed for fluorescence observation of the fluorescent light images and reflected light images displayed on the monitor 6 will have the prescribed brightness set by the operator. In this case, the CMD multiplication rate required during capturing of the green reflected light images and red reflected light images is 1/5 and 1/10 of the reference CMD multiplication rate used for fluorescent light images, as described previously. Therefore, the number of CMD input pulses when capturing green reflected light and red reflected light will respectively be 77 pulses (127 pulses - 50 pulses) and 55 pulses (127 pulses - 72 pulses), on the basis of the values set in the CCD sensitivity setting means 18.

Here, let it be assumed that the situation changes to close observation by moving the endoscope 2 nearer to the body tissue, and the CMD multiplication rate required to obtain a monochromatic fluorescent light image is 40 times (number of CMD input pulses = 115 pulses), according to the auto gain control. In this case, the number of CMD input pulses during capturing of green reflected light and red reflected light will be 65 pulses (115 pulses - 50 pulses) and 45 pulses (= 115 pulses - 72 pulses).

When 65 pulses and 45 pulses are input to the CMD magnification mechanism, the CMD multiplication rate will be 8 times and 4 times (see Fig. 9), and the ratio of the CMD multiplication rates for the respective wavelengths will be fluorescent light : green reflected light : red reflected light = 40 : 8 : 4 = 10 : 2 : 1, thereby maintaining the initially set value.

In this way, in the present embodiment, the CMD multiplication rate for the monochromatic fluorescent light image is subjected to auto gain control in such a manner that a uniform CCD 14 output signal level is obtained at all times, regardless of changes in the intensity of the subject, and the CMD multiplication rates when capturing green reflected light and red reflected light are subjected to auto gain control in such a manner that they maintain a uniform ratio with respect to the reference CMD multiplication rate used in capturing fluorescent light images. Moreover, in the image of the subject, the output signal characteristics and S/N characteristics illustrated in Fig. 4 and Fig. 5 are obtained. In particular, in regions of weak light, by increasing the number of input pulses to the charge multiplying mechanism CMD of the CCD 14, the output signal and the S/N ratio are greatly improved, and a high-quality fluorescent light observation image of suitable brightness is displayed on the monitor 6.

As described above, according to the first embodiment illustrated in Fig. 1 to Fig. 9, it is possible to perform capturing of light images of a plurality of specific wavelength regions by means of a single solid-state image pickup element, and moreover, when capturing images of fluorescent light and reflected light having very different intensities, in a special light mode (fluorescence observation mode), since a uniform ratio of multiplication rates for the charge multiplying mechanism CMD of the CCD 14 is maintained for respective wavelengths, it is possible to capture monochromatic images of fluorescent light and reflected light having appropriate brightness, and by synthesizing these images, a suitably bright, high-quality fluorescence observation image can be obtained.

Next, a second embodiment of the present invention is described.

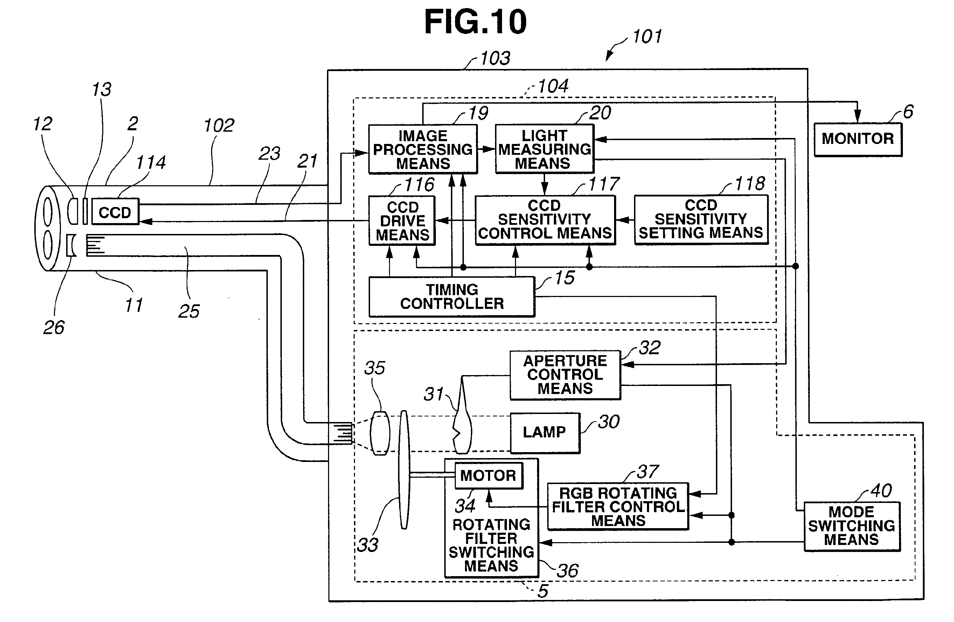

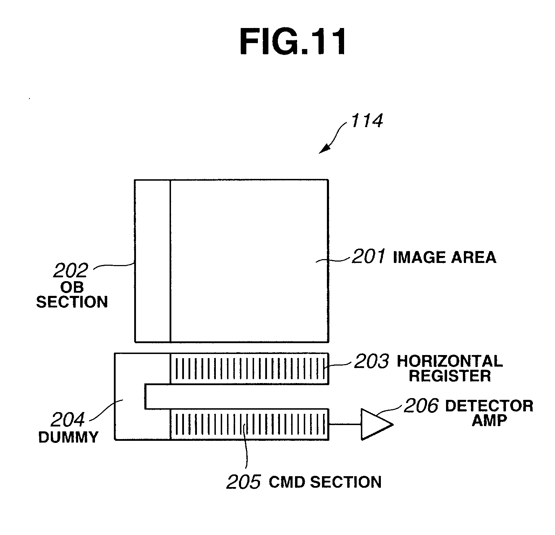

Fig. 10 is a block diagram showing the approximate composition of an endoscope device relating to the second embodiment of the present invention, and Fig. 11 is a block diagram of a CCD in the endoscope device according to the second embodiment illustrated in Fig. 10.

In the illustration in Fig. 10, constituent elements which are the same as those of the embodiment in Fig. 1 are similarly labelled and description thereof is omitted.

In Fig. 10, an endoscope device 101 comprises an endoscope 102 which built-in CCD 114, a processor 103 and the monitor 6.

The processor 3 contains a signal processing device 104 and light source device 5.

CCD drive means 116, CCD sensitivity control means 117 and CCD sensitivity setting mans 118 form electric charge multiplication rate setting means for setting the ratio of multiplication rates between light of a plurality of specific wavelength regions received by the CCD 14.

The solid-state image pickup element, CCD 114, comprises a charge multiplying mechanism CMD, and varies the charge multiplication rate by applying a pulsed high-field drive signal to the charge multiplying mechanism CMD.

The charge multiplication rate setting means sets the ratio of charge multiplication rates by changing the pulse voltage (CMD voltage) of the pulsed high-field drive signal.

As shown in Fig. 11, the CCD 114 is principally constituted by an image area 201, OB (optical black) section 202, horizontal register 203, dummy 204, charge multiplying mechanism (CMD section) 205 and detector amp 206.

The charge multiplying mechanism (CMD section) 205 is provided at post-step of the horizontal register and comprises a plurality of charge multiplying mechanisms CMD having the same number of stages as the number of horizontal register stages.

The CCD drive means 116 output drive signal to the CCD 114.

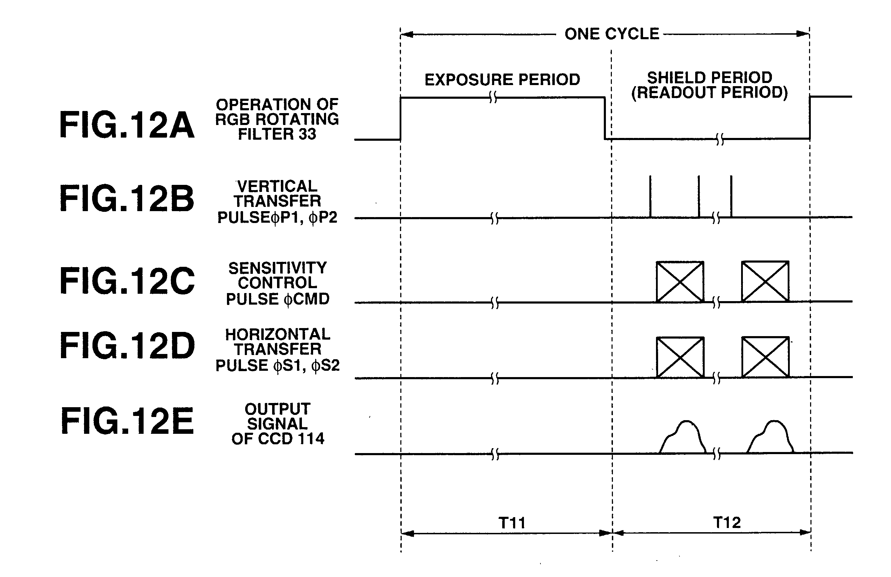

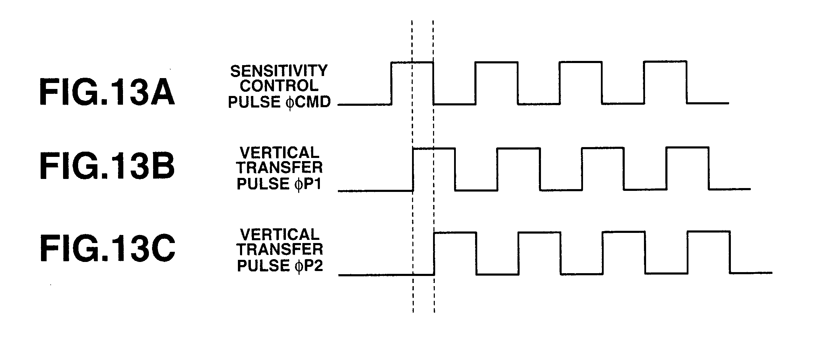

Fig. 12A to Fig. 12E and Fig. 13A to Fig. 13C are timing charts showing the relationship between a drive signal and CCD output signal in special light mode.

Fig. 12A shows the operation of an RGB rotating filter 33 in special light mode in the endoscope device 101 of the second embodiment illustrated in Fig. 10; Fig. 12B illustrates a first and second vertical transfer pulses φP1, φP2 supplied to the CCD 114 by the CCD drive means 116 in the endoscope device according to the second embodiment; Fig. 12C illustrates a sensitivity control pulse φCMD supplied to the CCD 114 in the endoscope device 101 according to the second embodiment illustrated in Fig. 10; Fig. 12D illustrates first and second horizontal transfer pulses φS1, φS2 supplied to the CCD 114 from the CCD drive means 116 in the endoscope device 101 according to the second embodiment illustrated in Fig. 10; Fig. 12E illustrates the output signal of the CCD 114 in the endoscope device 101 according to the second embodiment illustrated in Fig. 10; Fig. 13A shows an enlarged view of the time axis of the sensitivity control pulse φCMD shown in Fig. 12C, in the endoscope device 101 according to the second embodiment illustrated in Fig. 10; Fig. 13B shows an enlarged view of the time axis of the first vertical transfer pulse φP1 shown in Fig. 12B, in the endoscope device 101 according to the second embodiment illustrated in Fig. 10; and Fig. 13C shows an enlarged view of the time axis of the second vertical transfer pulse φP2 shown in Fig. 12B, in the endoscope device 101 according to the second embodiment illustrated in Fig. 10.

The sensitivity control pulse φCMD, the first and second vertical transfer pulses φP1, φP2, and the first and second horizontal transfer pulses φS1, φS2 are drive signals output from the CCD drive means 116 to the CCD 114.

Moreover, the sensitivity control pulse φCMD shown in Fig. 12C and Fig. 13A is a variable voltage pulse input from the CCD sensitivity control means 117.

As shown in Fig. 12A, in special light mode, the RGB rotating filter 33 performs one cycle in the exposure period T11 and shield period T12.

The time period T11 is the exposure period during which light from the lamp 30 is transmitted into the endoscope 102, and during this period T11, the CCD 114 accumulates signal charge on the image area 201 by photoelectrically converting the light incident on the light receiving surface thereof from the subject.

The shield period T12 is a shielding period in which the light from the lamp 30 is shut out and read out is performed at the CCD 114.

In time period T12, the sensitivity control pulse φCMD illustrated in Fig. 12C is supplied to the charge multiplying mechanism CMD of the CCD 114. In this state, the CCD 114 transfers the signal charge for one horizontal line accumulated in the image area 201 during period T11 to the horizontal register 203, in accordance with the first and second vertical transfer pulses φP1, φP2, and further transfers it to the horizontal register 203 and charge multiplying mechanism (CMD section) 205, in accordance with the first and second horizontal transfer pulses φS1, φS2, in such a manner that multiplication and transfer is repeated, one stage at a time, in the plurality of charge multiplying mechanisms CMD, thereby successively transmitting charge to the output amp section 206. The output amp section 206 of the CCD 114 converts the transferred charge to a voltage and outputs it as an output signal to the CCD 114 shown in Fig. 12E.

In the charge multiplying mechanism (CMD section) 205, focusing on the number of signal charges for one pixel, the charge number is gradually multiplied, little by little, each time it passes through one stage of the plurality of CMDs, until ultimately, a large multiplication is achieved. In other words, in the present embodiment, by altering the pulse voltage of the sensitivity control pulse φCMD supplied from the CCD drive means 116 to the charge multiplying mechanisms CMD, the desired sensitivity of the CCD 114 (CMD multiplication rate) is obtained.

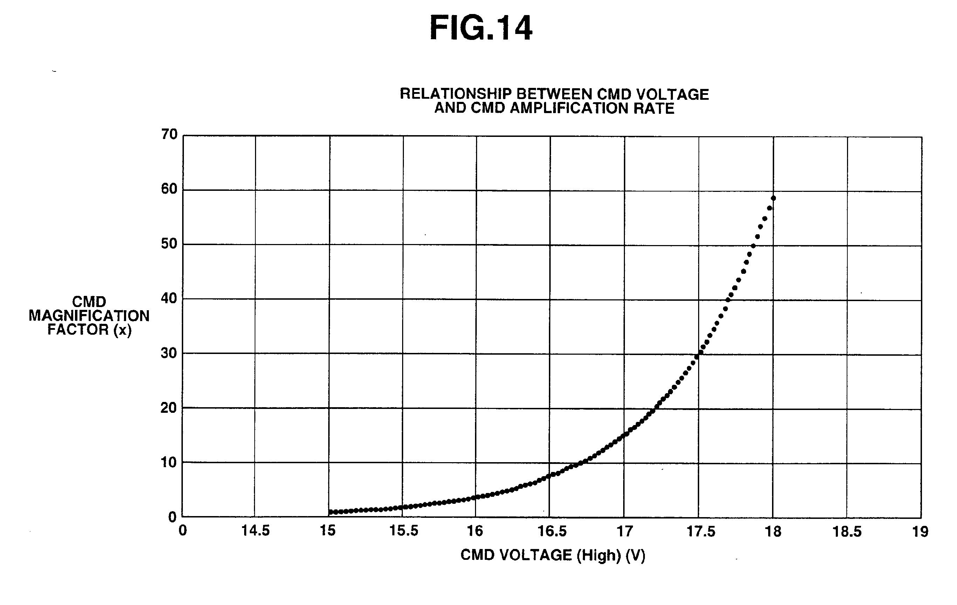

Next, one example of the relationship between the voltage supplied to the charge multiplying mechanism CMD of the CCD 114 and the CMD multiplication rate (signal charge multiplication rate by the CMD) is described with reference to Fig. 14.

Fig. 14 shows the relationship between CMD voltage and CMD multiplication rate with respect to CCD sensitivity, in the endoscope device 101 according to the second embodiment illustrated in Fig. 10.

In Fig. 14, the CCD 114 starts CMD multiplication when the voltage supplied to the charge multiplying mechanisms CMD exceeds a certain threshold value (in the present embodiment, 15V), and it has multiplication characteristics which rise exponentially with respect to increase in the voltage value.

Here, the graph in Fig. 14 shows the total multiplication characteristics of the plurality of CMDs, rather than the characteristics of one stage of the plurality of CMDs.

Moreover, in normal light mode, the sensitivity control pulse φCMD is not output to the CCD drive means 116 during time period T12.

If there is no sensitivity control pulse φCMD, or if the supplied voltage value is less than the threshold value, then the CCD 114 will have the same characteristics as the standard CCD sensitivity.

The main functions of the CCD sensitivity control means 117 are two: a CMD-AGC function and a CMD multiplication rate uniform ratio control function.

The CMD-AGC (auto gain control) function controls the CMD multiplication rate in such a manner that the output signal level from the CCD 114 is uniform with respect to change in the intensity incident on the light receiving surface of the CCD 114.

The CMD multiplication rate uniform ratio control function ensures that there is a uniform ratio of the CMD multiplication rates when capturing three wavelengths, namely, when capturing images corresponding to the filters 42a, 42b, 42c of spectral characteristics Ex1, Ex2, Ex3 of the RGB rotating filter 33.

Below, the CMD-AGC function of the CCD sensitivity control means 117 is described in detail.

When exercising the CMD-AGC function, the CCD sensitivity control means 117 inputs the average screen value of the monochromatic light image of a predetermined wavelength (in the present embodiment, the fluorescent light image), from the light measuring means 20, and compares this average screen value with a monitor brightness set as desired by the operator. The CCD sensitivity control means 117 calculates the voltage value of the sensitivity control pulse φCMD to be output from the CCD drive means 116 to the charge multiplying mechanisms CMD of the CCD 114, on the basis of the comparison result (magnitude relationship), and outputs a pulse of this voltage to the CCD drive means 116.

The equation for calculating the voltage of the sensitivity control pulse φCMD of the CCD sensitivity control means 117 is described below.

The relationship illustrated in Fig. 14 between the voltage of the sensitivity control pulse φCMD and the CMD multiplication rate charge multiplying mechanisms CMD in the CCD 114 can be expressed by Equation (4) below.

$$M(V) = D \cdot Exp\ (\beta(V\text{-}Vth)) \qquad (4)$$

M(V) is the CMD multiplication rate when the voltage of the sensitivity control pulse φCMD is V, Vth is the threshold value at which CMD multiplication starts, and D and β are intrinsic device constants.

According to these characteristics, when capturing images of a subject of a certain intensity, the average screen value of the image changes exponentially with increase or decrease in the voltage of the sensitivity control pulse φCMD.

The CCD sensitivity control means 117 is provided with an up/down counter.

The CCD sensitivity control means 117 compares the magnitude of the average screen value of the fluorescent light image and the prescribed monitor brightness set by the operator, and if the fluorescent light image is dimmer than the monitor brightness set by the operator, then the voltage of the sensitivity control pulse φCMD is increased by ΔV by the up/down counter, in order to increase the CMD multiplication rate, and at the next fluorescent light image capturing timing, the voltage value (V + ΔV) is output to the CCD drive means 116. Conversely, if the fluorescent light image is brighter than the monitor brightness set by the operator, then the voltage of the sensitivity control pulse φCMD is decreased by ΔV, and at the next fluorescent light image capturing timing, the voltage value (V - ΔV) is output to the CCD drive means 116.

Thereby, the CCD sensitivity control means 117 compares the average screen value of the fluorescent light image and the monitor brightness set by the operator and increases or decreases the voltage value, in such a manner that the average screen value of the fluorescent light matches the monitor brightness set by the operator, irrespective of change in the fluorescent light intensity of the subject.

The voltage of the sensitivity control pulse φCMD output by the CCD sensitivity control means 117 may be set to have a maximum limit and a minimum limit, and sensitivity control pulse φCMD voltage values within this range are output to the CCD drive means 116.

Below, the CMD multiplication rate uniform ratio control function of the CCD sensitivity control means 117 is described in detail.

When exercising the CMD multiplication rate uniform ratio control function, the CCD sensitivity control means 117 inputs the respective voltage values (differentials) corresponding to the ratio of CMD multiplication rates required for the remaining two wavelengths, with respect to the CMD multiplication rate of the reference wavelength (in the present embodiment, the fluorescent light image), from the CCD sensitivity setting means 118.

At the timing of capturing the reflected light images (2 images), the CCD sensitivity control means 117 respectively subtracts (or adds) the voltage value input from the CCD sensitivity setting means 118, from or to the voltage of the sensitivity control pulse φCMD output to the CCD drive means 116 when capturing fluorescent light images, and outputs the new voltage to the CCD drive means 116.

The CCD sensitivity setting means 118 sets the ratio of CMD multiplication rates for capturing the respective images corresponding to three wavelengths (Ex1, Ex2, Ex3) of the RGB rotating filter 33, in the special light mode (fluorescence observation).

The CCD sensitivity setting means 118 comprises two sets of setting means consisting of DIP switches, rotary DIP switches, or the like, which respectively set voltage values (differentials) corresponding to the ratio of CMD multiplication rates required for the remaining two wavelengths, with respect to the CMD multiplication rate of the reference wavelength (in the present embodiment, the fluorescent light images), and output the same to the CCD sensitivity control means 117.

The method of setting the sensitivity control pulse φCMD voltage values (differentials) in this case is described below.

Here, the relationship between the voltage of the sensitivity control pulse φCMD and the CMD multiplication rate in the charge multiplying mechanisms CMD of the CCD 14 is expressed by the aforementioned Equation (4), when the voltage of the sensitivity control pulse φCMD is V.

If the voltage of the sensitivity control pulse φCMD is (V - ΔV), then Equation (5) is obtained.

$$M(V - \Delta V) = D \cdot Exp\ (\beta\ (V - Vth - \Delta V)) \qquad (5)$$

From Equation (4) and Equation (5), the ratio between the CMD multiplication rates when the voltage of the sensitivity control pulse φCMD is V and (V - ΔV) is expressed by Equation (6).

$$M\ (V - \Delta V)\ /\ M\ (V)$$

$$= D \cdot Exp\ (\beta\ (V - Vth - \Delta V))\ /\ D \cdot Exp\ (\beta\ (Vth - V))$$

$$= Exp\ (-\beta\Delta V) \qquad (6)$$

From Equation (6), if the CMD multiplication rate for the reflected light is to be obtained at a uniform ratio with respect to the reference CMD multiplication rate of the fluorescent light image, then the voltage ΔV corresponding to Exp(-βΔV) should be subtracted from the voltage of the sensitivity control pulse φCMD when capturing the fluorescent light image. When the voltage is subtracted, a CMD multiplication rate that is lower than the reference CMD multiplication rate is obtained, and conversely, when the voltage is added, then a CMD multiplication rate that is greater than the reference CMD multiplication rate is obtained.

In contrast to the first embodiment illustrated in Fig. 1 to Fig. 9, according to the second embodiment illustrated in Fig. 10 to Fig. 14, the action of controlling the CMD multiplication rate by means of the number of pulses in the first embodiment is performed by means of voltage values in the second embodiment. Besides this, the action of the second embodiment is essentially the same as that of the first embodiment.

According to this second embodiment, the same merits as those of the first embodiment illustrated in Fig. 1 to Fig. 9 can be obtained, and moreover, the signal charge for all the pixels of the CCD 114 is multiplied by the same charge multiplying mechanism CMD, and hence there is little variation of the CMD multiplication rate and images of even higher quality than the first embodiment can be obtained.

The embodiments illustrated in Fig. 1 to Fig. 14 related to examples where one CCD is installed on the very end of the endoscope, but it is also possible to install two CCDs on the end of the endoscope and use the first CCD for the normal light mode and a second CCD for the special light mode. In this case, for example, CCD drive switching means consisting of a relay, or the like, is provided inside the endoscope, and the CCDs corresponding to the respective observation modes are driven in accordance with an observation mode switching signal from the processor.

Moreover, in the embodiments illustrated in Fig. 1 to Fig. 14, the CCD is mounted on the front end of the endoscope, but it is also possible to adopt a composition wherein the CCD is installed outside the endoscope, and images are obtained by provided image fibres inside the endoscope. In this case, the CCD may be fitted freely detachably with respect to the endoscope having image fibres, or it may be formed integrally with the endoscope.

Here, in the embodiments illustrated in Fig. 1 to Fig. 14, the fluorescent light and reflected light of very different intensities are captured at the same exposure time (accumulation period), and therefore the number of signal charges generated by the CCD differs very greatly for the fluorescent light and reflected light. Consequently, the ratio between the CMD multiplication rates for different wavelengths is very large, and hence the dynamic range of the CCD is small. In response to this, it is also possible to provide an electronic shutter mechanism in the CCD. By using an electronic shutter in the CCD and lengthening the exposure time (accumulation period) of the fluorescent light having low intensity, compared to the reflected light having high intensity, it is possible to reduce the ratio (differential) between the number of signal charges generated by the fluorescent light and the reflected light at the CCD, and hence the ratio (differential) between the CMD multiplication rates at different wavelengths can be reduced. Thereby, the dynamic range of the CCD can be increased.

Moreover, in the embodiments illustrated in Fig. 1 to Fig. 14, the three wavelengths in the special light mode are taken as those of fluorescent light, green reflected light and red reflected light, but the wavelength of the excitation light for fluorescence, the wave band of the excitation light, and the wavelength, central wavelength, half-width, transmissivity, and the like, of the reflected light, can be selected and combined in various ways.

Moreover, in the embodiments illustrated in Fig. 1 to Fig. 14, an up/down counter (not illustrated) is provided in the CCD sensitivity control means. A memory is provided in the up/down counter to store the number of sensitivity control pulses φCMD supplied to the charge multiplying mechanism CMD with respect to the reference wavelength (in the present embodiment, the fluorescent light image).

Moreover, in the embodiments illustrated in Fig. 1 to Fig. 14, the CCD sensitivity setting means forming the aforementioned charge multiplication rate setting means uses the wavelength of the fluorescent light image as the reference wavelength when setting the uniform ratio of the charge multiplication rates of the aforementioned solid-state image pickup element for a plurality of specific wavelength regions, but the wavelength of a reflected light image may also be used as the reference wavelength.

Having described the preferred embodiments of the invention referring to the accompanying drawings, it should be understood that the present invention is not limited to those precise embodiments and various changes and modifications thereof could be made by one skilled in the art without departing from the spirit of scope of the invention as defined in the appended claims.

**Claims**

1. An endoscope device (1, 101) for irradiating light of a plurality of specific wavelength regions onto a subject, in successive fashion, receiving an optical signal on the basis of the light irradiated onto said subject, by means of a solid-state image pickup element (14, 114) capable of changing the charge multiplication rate for multiplying the optical signal, and processing the output signal from said solid-state image pickup element (14, 114), by signal processing means (19);
   **characterized in** comprising charge multiplication rate setting means (16, 17, 18, 116, 117, 118) for setting the ratio of charge multiplication rates between said light of a plurality of specific wavelength regions received by said solid-state image pickup element (14, 114).

2. The endoscope device (1) according to claim 1, **characterized in that** said solid-state image pickup element (14) comprises a charge multiplying mechanism, to which a pulsed high-field drive signal is applied to change said charge multiplication rate; and
   said charge multiplication rate setting means (16, 17, 18) sets the ratio of said charge multiplication rates by changing the number of pulses of said pulsed high-field drive signal.

3. The endoscope device (101) according to claim 1, **characterized in that** said solid-state image pickup element (114) comprises a charge multiplying mechanism, to which a pulsed high-field drive signal is applied to change said charge multiplication rate; and
   said charge multiplication rate setting means (116, 117, 118) sets the ratio of said charge multiplication rates by changing the pulse voltage of said pulsed high-field drive signal.

**4.** The endoscope device (1) according to claim 1, **characterized in that** said solid-state image pickup element (14) comprises a charge multiplying mechanism, to which a plurality of pulsed drive signals are applied to change said charge multiplication rate; and

said charge multiplication rate setting means (16, 17, 18) sets the ratio of said charge multiplication rates by changing the number of pulses of said plurality of pulsed drive signals.

**5.** The endoscope device (1) according to claim 1, **characterized in that** said solid-state image pickup element (14) is provided in an endoscope (2) and comprises a charge multiplying mechanism, to which a plurality of pulsed drive signals are applied to change said charge multiplication rate and thereby the sensitivity being made changeable; and

said charge multiplication rate setting means (16, 17, 18) sets the ratio of charge multiplication rates between said light of a plurality of specific wavelength regions to a uniform ratio, by changing the number of pulses of said plurality of pulsed drive signals.

**6.** The endoscope device (101) according to claim 1, **characterized in that** said solid-state image pickup element (114) is provided in an endoscope (102) and comprises a charge multiplying mechanism, to which the voltage of a pulsed drive signal is applied to change said charge multiplication rate and thereby the sensitivity being made changeable; and

said charge multiplication rate setting means (116, 117, 118) sets the ratio of charge multiplication rates between said light of a plurality of specific wavelength regions to a uniform ratio, by changing the voltage of said pulsed drive signal.

**7.** The endoscope device (1, 101) according to claim 1, **characterized in that** said light of a plurality of specific wavelength regions comprises excitation light for fluorescence and illumination light for reflected light.

**8.** The endoscope device (1, 101) according to claim 1, **characterized in that** said light of a plurality of specific wavelength regions comprises blue excitation light for fluorescence and narrow band light in green and red regions for reflected light.

**9.** The endoscope device (1, 101) according to claim 1, **characterized in that** said signal processing means (19) sets the white balance coefficient for said light of a plurality of specific wavelength regions.

**10.** The endoscope device (1, 101) according to claim 1, **characterized in that** further comprises mode switching means (40) switching observation modes between a normal light mode for observation by means of normal light and a special light mode for observation by means of said light of a plurality of specific wavelength regions.

**11.** The endoscope device (101) according to claim 3, **characterized in that** said solid-state image pickup element (114) further comprises a horizontal register, and said charge multiplying mechanism is provided at the post-step of this horizontal register.

# FIG.1

1 ENDOSCOPE DEVICE

3 PROCESSOR

13 EXCITATION LIGHT CUTOFF FILTER

2 ELECTRONIC ENDOSCOPE

12 OBJECTIVE LENS

10 INSERTABLE SECTION

14

CCD

23 21

11

26 ILLUMINATION LENS

25 LIGHT GUIDE

4 SIGNAL PROCESSING DEVICE

19 IMAGE PROCESSING MEANS

20 LIGHT MEASURING MEANS

6 MONITOR

16 CCD DRIVE MEANS

17 CCD SENSITIVITY CONTROL MEANS

18 CCD SENSITIVITY SETTING MEANS

15 TIMING CONTROLLER

35 CONDENSER LENS

32 APERTURE CONTROL MEANS

31

30 LAMP

34 MOTOR

37 RGB ROTATING FILTER CONTROL MEANS

40 MODE SWITCHING MEANS

33 RGB ROTATING FILTER

36 ROTATING FILTER SWITCHING MEANS

5 LIGHT SOURCE DEVICE

EP 1 294 186 A2

EP 1 294 186 A2

**FIG.2A** — OPERATION OF RGB ROTATING FILTER 33

**FIG.2B** — VERTICAL TRANSFER PULSE $\phi$P1

**FIG.2C** — VERTICAL TRANSFER PULSE $\phi$P2

**FIG.2D** — SENSITIVITY CONTROL PULSE $\phi$CMD

**FIG.2E** — HORIZONTAL TRANSFER PULSE $\phi$S1

**FIG.2F** — HORIZONTAL TRANSFER PULSE $\phi$S2

**FIG.2G** — OUTPUT SIGNAL OF CCD 14

ONE CYCLE

EXPOSURE PERIOD

SHIELD PERIOD (READOUT PERIOD)

T1  T2  T3

# FIG.3

**RELATIONSHIP BETWEEN NUMBER OF CMD PULSES AND CMD MAGNIFICATION FACTOR**

CMD MAGNIFICATION FACTOR (x)

NUMBER OF CMD PULSES

EP 1 294 186 A2

# FIG.4

OUTPUT SIGNAL (mV)
(PROCESSOR
OUTPUT STAGE)

CMD
MAGNIFICATION
x 10

714mV (100IRE)

CMD
MAGNIFICATION
x 3

CMD
MAGNIFICATION
x 1

INTENSITY OF SUBJECT (a.u.)

EP 1 294 186 A2

# FIG.5

S/N (dB)
(PROCESSOR
OUTPUT STAGE)

INTENSITY OF SUBJECT (a.u.)

CMD
MAGNIFICATION
FACTOR x 10

CMD
MAGNIFICATION
FACTOR x 1

CMD
MAGNIFICATION
FACTOR x 3

EP 1 294 186 A2

# FIG.6

# FIG.7

# FIG.8

FIG.9

RELATIONSHIP BETWEEN NUMBER OF CMD PULSES AND CMD MAGNIFICATION FACTOR

# FIG.10

MONITOR 6

IMAGE PROCESSING MEANS 19

LIGHT MEASURING MEANS 20

CCD 114

CCD DRIVE MEANS 116

CCD SENSITIVITY CONTROL MEANS 117

CCD SENSITIVITY SETTING MEANS 118

TIMING CONTROLLER 15

APERTURE CONTROL MEANS 32

LAMP 30

MOTOR 34

RGB ROTATING FILTER CONTROL MEANS 37

ROTATING FILTER SWITCHING MEANS 36

MODE SWITCHING MEANS 40

EP 1 294 186 A2

# FIG.11

114

201 IMAGE AREA

202 OB SECTION

203 HORIZONTAL REGISTER

206 DETECTOR AMP

204 DUMMY

205 CMD SECTION

**FIG.12A** OPERATION OF RGB ROTATING FILTER 33

**FIG.12B** VERTICAL TRANSFER PULSE φP1, φP2

**FIG.12C** SENSITIVITY CONTROL PULSE φCMD

**FIG.12D** HORIZONTAL TRANSFER PULSE φS1, φS2

**FIG.12E** OUTPUT SIGNAL OF CCD 114

ONE CYCLE

EXPOSURE PERIOD

SHIELD PERIOD (READOUT PERIOD)

T11

T12

EP 1 294 186 A2

**FIG.13A**  SENSITIVITY CONTROL PULSE φCMD

**FIG.13B**  VERTICAL TRANSFER PULSE φP1

**FIG.13C**  VERTICAL TRANSFER PULSE φP2

EP 1 294 186 A2

# FIG.14

RELATIONSHIP BETWEEN CMD VOLTAGE
AND CMD AMPLIFICATION RATE

CMD
MAGNIFICATION
FACTOR (x)

CMD VOLTAGE (High) (V)

EP 1 294 186 A2